# EUROPÄISCHE PATENTSCHRIFT

(11) **EP 1 379 497 B1**
(45) Veröffentlichungstag und Bekanntmachung des Hinweises auf die Patenterteilung: **17.01.2007**
(21) Anmeldenummer: 02706782.6
(22) Anmeldetag: 23.03.2002
(51) Int. Cl.: C07C 317/24, C07C 49/84, A01N 35/06, A01N 41/10

(54) **DERIVATE VON BENZOYLCYCLOHEXANDIONEN UND IHRE VERWENDUNG ALS HERBIZIDE**
DERIVATIVES OF BENZOYLCYCLOHEXANEDIONE AND THEIR USE AS HERBICIDES
DERIVES DE BENZOYLCYCLOHEXANEDIONES ET LEUR UTILISATION EN TANT QU'HERBICIDES

(30) Priorität: 07.04.2001 DE 10117503
(43) Veröffentlichungstag der Anmeldung: 14.01.2004
(73) Patentinhaber: Bayer CropScience GmbH, 65929 Frankfurt/Main (DE)
(72) Erfinder: SEITZ, Thomas, 68519 Viernheim (DE); VAN ALMSICK, Andreas, 61184 Karben (DE); WILLMS, Lothar, 65719 Hofheim (DE); AULER, Thomas, 42799 Leichlingen (DE); BIERINGER, Hermann, 65817 Eppstein (DE); MENNE, Hubert, 65719 Hofheim Ts. (DE)
(86) Internationale Anmeldenummer: PCT/EP2002/003290
(87) Internationale Veröffentlichungsnummer: WO 2002/081434

(56) Entgegenhaltungen:
- EP-A- 0 791 572
- US-A- 4 780 127

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte Benzoylcyclohexandione herbizide Eigenschaften besitzen. So sind aus EP-A 0 319 075, WO 92/07837 und WO 96/22958 Benzoylcyclohexandione mit einem Haloalkoxy-Rest in 3-Position des Phenylrings bekannt. In EP-A 0 563 817 werden Salze von Benzoylcyclohexandionen beschrieben, die ebenfalls in 3-Position des Phenylrings einen Haloalkoxy-Rest tragen. WO 91/05470, WO 92/13833 und US 4,780,127 nennen Benzoylcyclohexandione, die in 3-Position des Phenylrings unsubstituiert oder durch verschiedene Reste, darunter Trifluormethoxy, substituiert sein können. Das letztgenannte Dokumente nennt darüberhinaus die Verbindungen 2-(3-(2,3-Di-brompropoxy)-2-chlor-4-ethylsulfonylbenzoyl)-1,3-cyctohexandion und 2-(2-Chlor-3-(3-chlorpropoxy)-4-ethylsulfonylbenzoyl)-1,3-cyclohexandion.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den im dem Stand der Technik offenbarten Verbindungen - verbesserten herbiziden Eigenschaften.

Es wurde nun gefunden, daß Derivate von Benzoylcyclohexandionen, deren Phenylring in 3- Position durch ausgewählte Reste aus der Gruppe (C₁-C₄)-Halogenalkoxy substituiert ist, als Herbizide besonders gut geeignet sind. Ein Gegenstand der vorliegenden Erfindung sind daher Verbindungen der Formel (I) oder deren Salze 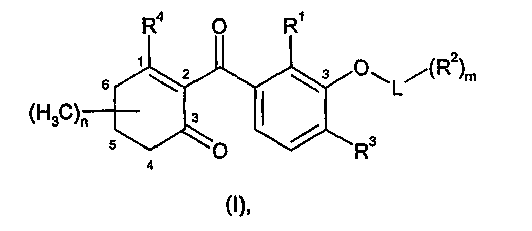 worin
- L: eine gesättigte Kohlenstoffkette mit 1, 2, 3 oder 4 Kohlenstoffatomen;
- R¹: Iod, Brom, Chlor, Fluor, (C₁-C₄)-Alkyl, (C₁-C₄)-Alkylthio, (C₁-C₄)-Alkoxy oder (C₁-C₄)-Halogenalkyl;
- R²: Brom, Chlor, Iod oder Fluor;
- R³: Brom, Chlor, Fluor, Cyano, Nitro, (C₁-C₄)-Alkyl, Methylsulfonyl oder Ethylsulfonyl;
- R⁴: OR⁵ oder SR⁵;
- R⁵: Wasserstoff, (C₁-C₄)-Alkyl, (C₂-C₄)-Alkenyl,(C₂-C₄)-Alkinyl, Benzyl, Phenyl;
- m: eine ganze Zahl von 1 bis 9 und
- n: 0, 1, oder 2 bedeuten,
mit der Maßgabe, daß
a) der Rest O - L - (R²)ₘ nicht für Trifluormethoxy stehen soll, und
b) die Verbindungen 2-(3-(2,3-Di-brompropoxy)-2-chlor-4-ethylsulfonylbenzoyl)-1,3-cyclohexandion und 2-(2-Chlor-3-(3-chlorpropoxy)-4-ethylsulfonylbenzoyl)-1 ,3-cyclohexandion
von obiger Definition nicht umfaßt sein sollen.

Für den Fall, daß R⁴ für OR⁵ steht, und R⁵ für Wasserstoff steht, können die erfindungsgemäßen Verbindungen der Formel (I) in Abhängigkeit von äußeren Bedingungen, wie Lösungsmittel und pH-Wert, in unterschiedlichen tautomeren Strukturen auftreten:

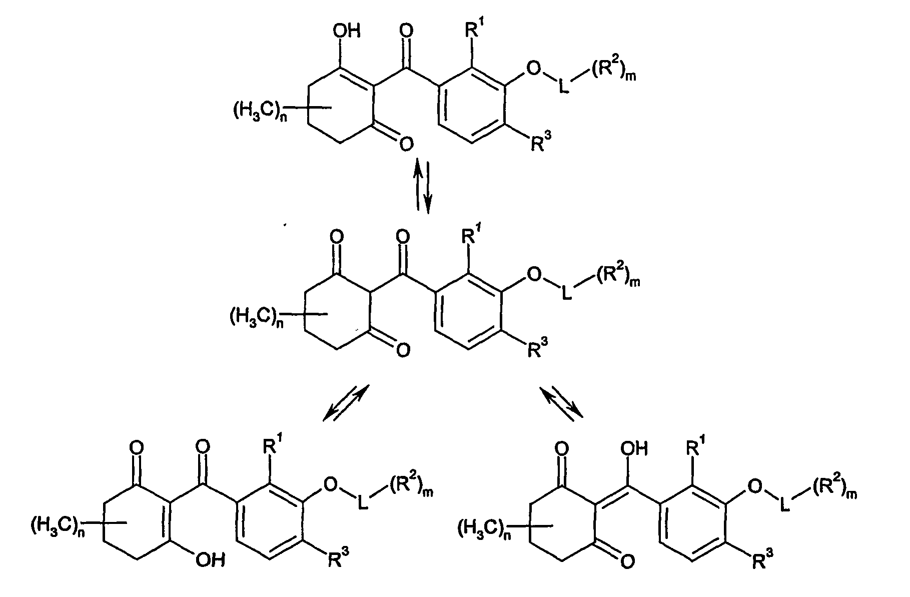

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Alkali- und Erdalkalimetallen, wie Lithium, Natrium, Kalium, Magnesium und Calcium, sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Die gesättigte Kohlenstoffkette L kann ebenfalls, je nach Anzahl ihrer Kohlenstoffatome geradkettig oder verzweigt sein. Die daran gebundenen m Atome der Gruppe Brom, Chlor und Fluor können sich an beliebiger Position dieser Kette befinden.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Als vorteilhaft haben sich Verbindungen der Formel (I) erwiesen, die nicht als Salz vorliegen.

Ebenfalls als vorteilhaft haben sich solche Verbindungen erwiesen, bei denen die Substituenten R¹ und R³ dieselbe Bedeutung haben.

Bevorzugt sind Verbindungen der allgemeinen Formel (I) worin
- R²: Brom, Chlor oder Fluor bedeutet.

Ebenfalls bevorzugt sind Verbindungen der allgemeinen Formel (I), worin
- L: eine gesättigte Kohlenstoffkette mit 1, 2 oder 3 Kohlenstoffatomen;
- R¹: Brom, Chlor, Fluor, Methyl, Methylthio, Methoxy oder Trifluormethyl;
- R³: Brom, Chlor, Fluor, Methylsulfonyl oder Ethylsulfonyl und
- m: eine ganze Zahl von 1 bis 7 bedeuten.

Besonders bevorzugt sind Verbindungen der allgemeinen Formel (I), worin
- R²: Chlor oder Fluor und
- R³: Chlor, Fluor, Methylsulfonyl oder Ethylsulfonyl bedeuten.

Ganz besonders bevorzugt sind Verbindungen der allgemeinen Formel (I), worin
- R¹: Brom oder Chlor;
- R⁵: Wasserstoff und
- n: 0 bedeuten.

Ebenfalls ganz besonders bevorzugt sind Verbindungen der allgemeinen Formel (I), worin R¹ für Chlor steht.

Eine weitere Gruppe ganz besonders bevorzugter Verbindungen der allgemeinen Formel (I) sind solche, worin R⁴ für OR⁵ steht.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁵ für Wasserstoff steht, können beispielsweise nach der in Schema 1 angegebenen Methode durch basenkatalysierte Umsetzung eines Benzoesäurehalogenids (III) mit einem Cyclohexandion (II) in Anwesenheit einer Cyanid-Quelle hergestellt werden. Solche Methoden sind beispielsweise in EP-A 0 186 117 beschrieben.

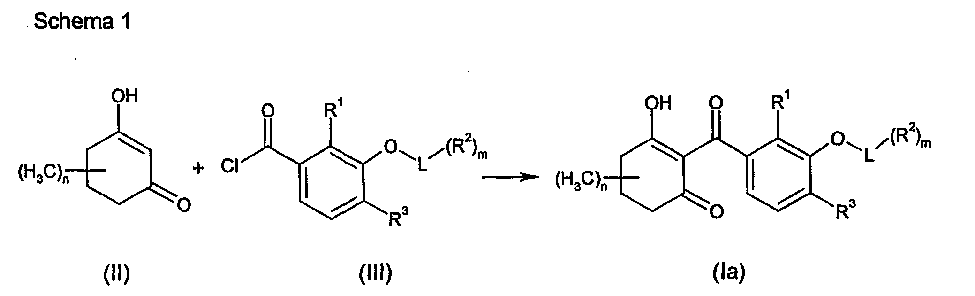

Erfindungsgemäße Verbindungen, in denen R⁵ eine andere Bedeutung als Wasserstoff hat, werden gemäß Schema 2 zweckmäßigerweise aus den nach Schema 1 erhältlichen Verbindungen durch Halogenierung mit einem geeigneten Halogenierungsmittel und darauf folgender basenkatalysierter Umsetzung mit einem Alkohol R⁵-OH oder Thiol R⁵-SH hergestellt. Solche Methoden sind beispielsweise aus J. Fluorine Chem. 66 (1984) 1, 39-46 und Khim. Farm. Zh. 30 (1996) 2, 27-30 bekannt.

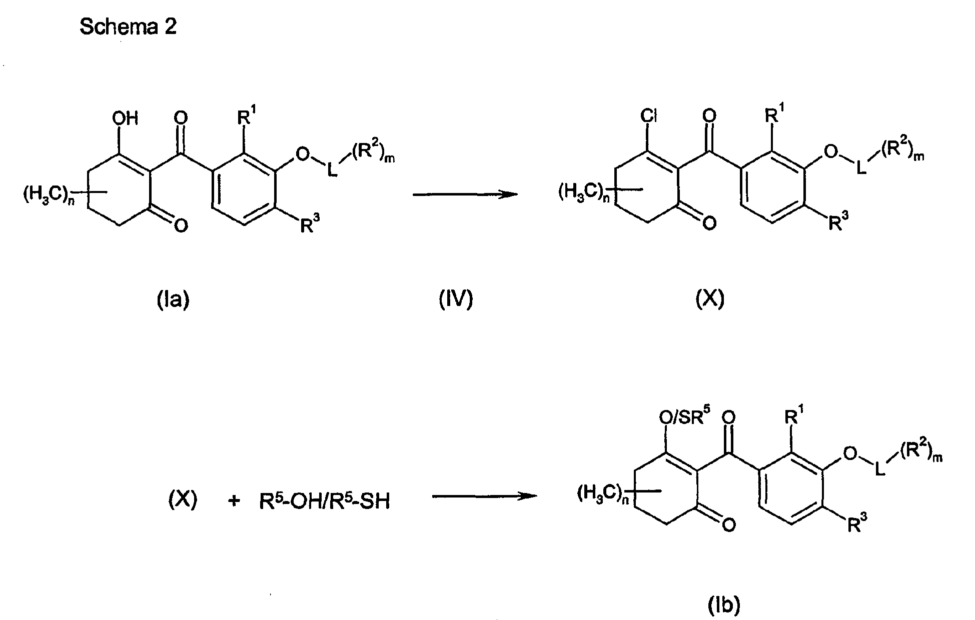

Die in obigen Schemata verwendeten Ausgangsverbindungen sind entweder käuflich oder nach an sich bekannten Methoden herstellbar. Die Benzoylchloride (III) können beispielsweise aus den entsprechenden Benzoesäuren oder Benzoesäureestern der Formel (IIIa), worin T für Hydroxy oder Alkoxy steht, hergestellt werden. Diese Benzoesäuren und Benzoesäureester der Formel (IIIa) können beispielsweise gemäß Schema 3 aus den Hydroxy-Derivaten (V) durch Umsetzung mit Verbindungen der Formel (VI), worin M¹ für eine Abgangsgruppe wie Halogen, Mesyl, Tosyl, Triflat und Nonaflat steht, hergestellt werden. Solche Methoden sind z.B. aus Houben-Weyl Band 6/3, S. 54 bis 69, Band 9, S. 103 bis 115 und Band 11, S. 97 bekannt.

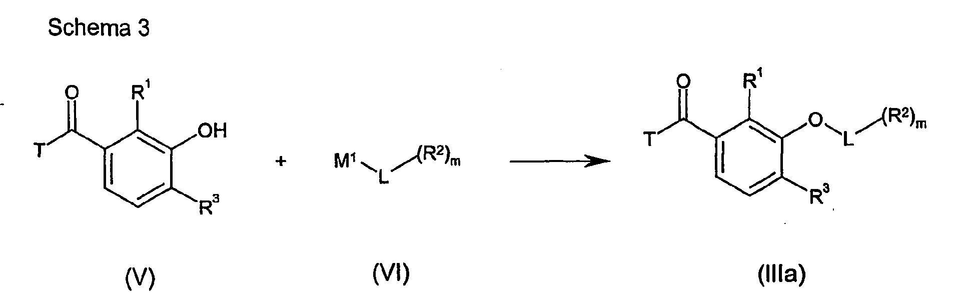

Verbindungen der Formel (IIIa) können gemäß Schema 4 auch durch Umsetzung von Verbindungen der Formel (V) mit Olefinen der Formel (VII), worin L' für eine gesättigte Kohlenstoffkette mit 1 bis 2 Kohlenstoffatomen und R^{2a} und R^{2b} jeweils für Chlor oder Fluor stehen, hergestellt werden. Solche Methoden sind z. B. in Zh. Org. Khim. 27 (1991) 4, 781-788 beschrieben.

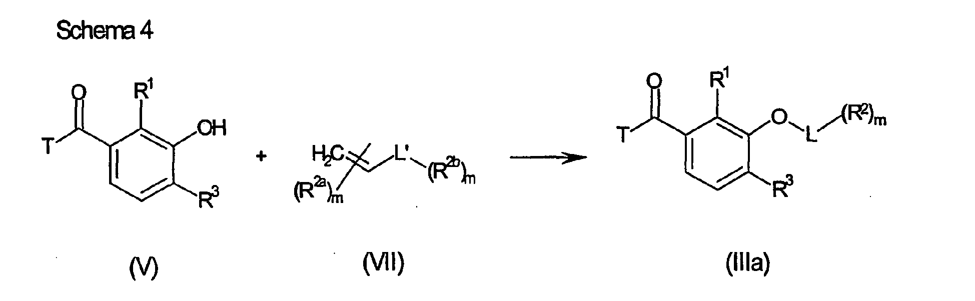

Verbindungen der Formel (IIIa), worin T für Hydroxy oder Alkoxy steht, können gemäß Schema 5 durch Umsetzung von Verbindungen der Formel (VIII), worin M¹ für eine Abgangsgruppe wie Halogen, Mesyl, Tosyl, Triflat und Nonaflat steht, hergestellt werden. Solche Methoden sind beispielsweise WO 98/42648, Houben Weyl Band 6/3, S. 75 bis 78, Band 9, S. 103 bis 105 bekannt.

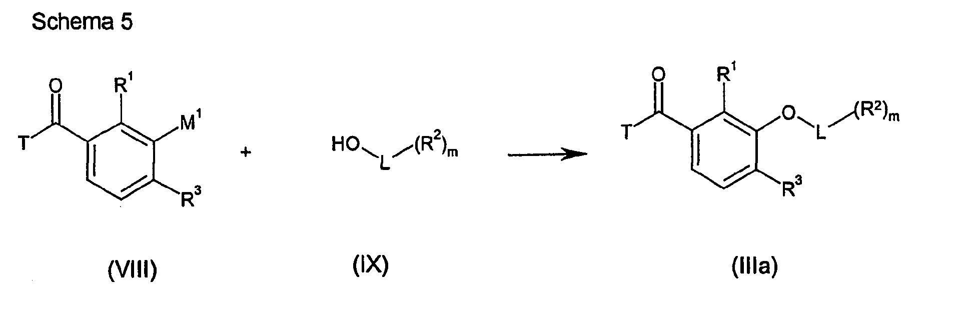

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Amaranthus retroflexus, Avena sp., Echinochloa sp., Cyperus serotinus, Lolium multiflorum, Setaria viridis, Sagittaria pygmaea, Scirpus juncoides, Sinapis sp. und Stellaria media.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Weizen, Mais und Reis auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vor-gegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

Die Herstellung der Ausgangsverbindung 2,4-Dibrom-3-hydroxybenzoesäureethylester erfolgte gemäß US 5,026,896, die Herstellung von 2-Chlor-3-hydroxy-4-ethylsulfonyl-benzoesäure erfolgte gemäß US 709,006. Perfluorbutansulfonsäure-(2,2,3,3-tetrafluorpropyl)-ester und Perfluorbutansulfonsäure-(2,2,2-trifluormethyl)-ester wurden gemäß J. Org. Chem. USSR 14 (1978) 808-809 hergestellt.

Die Abkürzung RT steht für Raumtemperatur.

### 1. Herstellung von 2-(2-Chlor-3-(2,2,3,3-tetrafluorpropyloxy)-4-ethylsulfonylbenzoyl)-cyclohexan-1,3-dion

### Schritt 1: 2-Chlor-3-hydroxy-4-ethylsulfonyl-benzoesäuremethylester

33.0 g (124.7 mmol) 2-Chlor-3-hydroxy-4-ethylsulfonyl-benzoesäure wurden in 1300 ml Methanol gelöst. Es wurden 174 ml (3263 mmol) konz. H₂SO₄ zugetropft, und die Mischung wurde 5 h unter Rückfuß erhitzt. Das Reaktionsgemisch wurde eingeengt, und der Rückstand wurde in Methylenchlorid aufgenommen. Es wurde mit Waser gewaschen, über Na₂SO₄ getrocknet und vollständig eingeengt. Man erhielt 2-Chlor-3-hydroxy-4-ethylsulfonyl-benzoesäuremethylester als gelbes, zähes Öl.
Ausbeute: 28.23 g (81 % der Theorie)
¹H-NMR:
δ [CDCl3] 1.32 (t, 3H), 3.24 (q, 2H), 3.96 (s, 3H), 7.38 (d, 1 H), 7.65 (d, 1H)
R_{f} (Essigester): 0.45

### Schritt 2: 2-Chlor-3-(2,2,3,3-tetrafluor-propyloxy)-4-ethylsulfonylbenzoesäuremethylester

1.200 g (4.3 mmol) Kalium-tert.-butylat und 1.917 g (4.3 mmol) Perfluorbutansulfonsäure-(2,2,3,3-tetrafluorpropyl)-ester wurden in 30 ml DMF vorgelegt. Bei RT wurden 1.200 g (4.3 Mol) 2-Chlor-3-hydroxy-4-ethylsulfonylbenzoesäuremethylester zugegeben und dann für 7 h auf 120°C erhitzt. Anschliessend wurde auf Wasser gegeben und mit Diisopropylether extrahiert. Die vereinigten organischen Phasen wurden mit Wasser gewaschen, über Na₂SO₄ getrocknet und eingeengt. Trocknen im Ölpumpenvakuum ergab 2-Chlor-3-(2,2,3,3-tetrafluor-propyloxy)-4-ethylsulfonyl-benzoesäuremethylester als braunes Öl. Ausbeute: 1.60 g (95% der Theorie)
¹H-NMR: δ [CDCl3] 1.27 (t, 3H), 3.43 (q, 2H), 4.00 (s, 3H),4.66 (m, 2H), 6.07 (m, 1 H), 7.76 (d, 1 H), 7.96 (d, 1 H)
R_{f} (Essigester): 0.73

### Schritt 3: 2-Chlor-3-(2,2,3,3-tetrafluor-propyloxy)-4-ethylsulfonyl-benzoesäure

1.600 g (4.07 mmol) 2-Chlor-3-(2,2,3,3-tetrafluor-propyloxy)-4-ethylsulfonylbenzoesäuremethylester wurden in einer Mischung aus 20 ml THF und 20 ml Wasser gelöst und mit 0.218 g (5.50 mmol) Natriumhydroxid versetzt. Die Mischung wurde 12 h bei RT gerührt und eingeengt. Der Rückstand wurde in Wasser aufgenommen und mit 6 N HCl versetzt. Der ausgefallene Niederschlag wurde abgesaugt und getrocknet. Es wurde 2-Chlor-3-(2,2,3,3-tetrafluor-propyloxy)-4-ethylsulfonyl-benzoesäure in Form eines weißen Feststoffes erhalten.
Ausbeute: 1.45 g (89% der Theorie)
¹H-NMR: δ [d6-DMSO] 1.11 (t, 3H), 3.49 (q, 2H), 1.62 (m, 4H),4.62 (m, 2H), 6.76 (m, 1 H), 7.80 (d, 1 H), 7.89 (d, 1 H)
Festpunkt: 163-166°C

### Schritt 4: ((3-Oxo-1-cyclohexenyl)-2-chlor-3-(2,2,3,3-tetrafluor-propyloxy)-4-ethylsulfonyl)-benzoat

0.710 g (1.90 mmol) 2-Chlor-3-(2,2,3,3-tetrafluor-propyloxy)-4-ethylsulfonylbenzoesäure, 0.231 g (2.10 mmol) Cyclohexan-1,3-dion, 0.403 g (2.10 mmol) N'-(3-Dimethylaminopropyl)-N-ethylcarbodümid Hydrochlorid und 0.002 g Dimethylaminopyridin wurden in 10 ml CH₂Cl₂ 10 h bei RT gerührt. Anschliessend wurde mit CH₂Cl₂ verdünnt und mit 0.5 N HCl, mit Wasser, mit gesättigter NaHCO₃-Lösung und wieder mit Wasser gewaschen. Nach Trocknen der vereinigten organischen Phasen über Na₂SO₄ und vollständigem Einengen wurde ((3-Oxo-1-cyclohexenyl)-2-chlor-3-(2,2,3,3-tetrafluor-propyloxy)-4-ethylsulfonyl)-benzoat in Form eines braunen Harzes erhalten, das für die Folgeumsetzung ausreichend rein war.
Ausbeute: 0.720 g
¹H-NMR:
δ [CDCl3] 1.27 (t, 3H), 2.16 (m, 2H), 2.49 (m, 2H), 2.70 (m, 2H), 3.44 (q,2H), 4.66 (m, 2H), 6.05 (m, 1 H), 6.08 (s, 1 H), 7.86 (d, 1 H), 8.02 (d, 1H)
R_{f} (Essigester): 0.65

### Schritt 5: 2-(2-Chlor-3-(2,2,3,3-tetrafluor-propyloxy)-4-ethylsulfonyl-benzoyl)-cyclohexan-1,3-dion

0.690 g (1.50 mmol) ((3-Oxo-1-cyclohexenyl)-2-chlor-3-(2,2,3,3-tetrafluor-propyloxy)-4-ethylsulfonyl)-benzoat wurde in 10 ml Acetonitril gelöst. Es wurden 3 Tropfen Acetoncyanhydrin sowie 0.258 g (2.60 mmol) NEt₃ zugegeben. Die Mischung wurde 2 h bei RT gerührt, worauf hin 0.044 g (0.70 mmol) KCN zugegeben wurden. Nach weiteren 10 h bei RT wurde vollständig eingeengt, der Rückstand in Wasser aufgenommen und mit 6 N Salzsäure versetzt. Anschliessend wurde mit CH₂Cl₂ extrahiert. Nach Trocknen der vereinigten organischen Phasen über Na₂SO₄, vollständigem Einengen und Chromatographie an reversed-phase-Kieselgel (Laufmittel: Acetonitril/Wasser-Gradient) erhielt man 2-(2-Chlor-3-(2,2,3,3-tetrafluorpropyloxy)-4-ethylsulfonyl-benzoyl)-cyclohexan-1,3-dion in Form eines farblosen, zähen Öls.
Ausbeute: 0.198 g (28% der Theorie)
¹H-NMR: δ [CDCl3] 1.31 (t, 3H), 2.09 (m, 2H), 2.59 (m, 2H), 2.84 (m, 2H), 3.40 (q, 2H), 4.64 (m, 2H), 6.06 (m, 1 H), 7.16 (d, 1H), 7.95 (d, 1H)
R_{f} (Essigester): 0.23

### 2. Herstellung von 2,4-Dibrom-3-(2,2,2-trifluorethoxy)-cyclohexan-1,3-dion

### Schritt 1: 2,4-Dibrom-3-(2,2,2-trifluorethoxy)-benzoesäureethylester,

0.416 g (3.70 mmol) Kalium-tert.-butylat und 1.200 g (3.70 mmol) 2,4-Dibrom-3-hydroxy-benzoesäureethylester wurden in 15 ml DMF vorgelegt. Bei RT wurden 1.490 g (3.70 mmol) Perfluorbutansulfonsäure-(2,2,2-trifluorpeopyl)-ester zugegeben, und die Mischung wurde für 6 h auf 120°C erhitzt. Anschliessend wurde auf Wasser gegeben und mit Diisopropylether extrahiert. Die vereinigten organischen Phasen wurden mit Wasser gewaschen, über Na₂SO₄ getrocknet und vollständig eingeengt. Trocknen im Ölpumpenvakuum ergab 2,4-Dibrom-3-(2,2,2-trifluorethoxy)-benzoesäureethylester als braunes Öl.
Ausbeute: 1.42 g (79% der Theorie)
¹H-NMR: δ [CDCl3] 1.20 (t, 3H), 4.32-4.46 (m, 2H + 2H), 7.45 (d, 1H), 7.60 (d, 1 H)
R_{f} (Essigester): 0.82

### Schritt 2: 2,4-Dibrom-3-(2,2,2-trifluorethoxy)-benzoesäure

1.380 g (3.40 mmol) 2,4-Dibrom-3-(2,2,2-trifluorethoxy)-benzoesäureethylester wurden in einer Mischung aus 15 ml THF und 15 ml Wasser gelöst und mit 0.150 g (3.47 mmol) NaOH versetzt. Die Mischung wurde 12 h bei RT gerührt und vollständig eingeengt. Der Rückstand wurde in Wasser aufgenommen und mit 6 N HCl versetzt. Der ausgefallene Niederschlag wurde abgesaugt und getrocknet. Es wurde 2-Chlor-2,4-Dibrom-3-(2,2,2-trifluorethoxy)-benzoesäure in Form eines weißen Feststoffes erhalten.
Ausbeute: 1.01 g (79% der Theorie)
¹H-NMR: δ [d6-DMSO] 4.6 (m, 2H), 7.46 (d, 1H), 7.80 (d, 1 H)
Festpunkt: 148-151°C

### Schritt 3: ((3-Oxo-1-cyclohexenyl)-2,4-dibrom -3-(2,2,2-trifluorethoxy))-benzoat

0.490 g (1.30 mmol) 2,4-Dibrom-3-(2,2,2-trifluorethoxy)-benzoesäure, 0.160 g (1.40 mmol) Cyclohexan-1,3-dion, 0.279 g (1.40 mmol) N'-(3-Dimethylaminopropyl)-N-ethylcarbodümid Hydrochlorid und 0.002 g Dimethylaminopyridin wurden in 10 ml CH₂Cl₂ 30 h bei RT gerührt. Anschliessend wurde mit CH₂Cl₂ verdünnt und mit 0.5 N HCl, mit Wasser, mit gesättigter NaHCO₃-Lösung und wieder mit Wasser gewaschen. Nach Trocknen der vereinigten organischen Phasen über Na₂SO₄ und vollständigem Einengen wurde ((3-Oxo-1-cyclohexenyl)-2,4-dibrom -3-(2,2,2-trifluorethoxy))-benzoat in Form eines gelben Harzes erhalten, das für die Folgeumsetzung ausreichend rein war.
Ausbeute: 0.37 g
¹H-NMR: δ [CDCl3] 2.14 (m, 2H), 2.46 (m, 2H), 2.70 (m, 1H), 4.42 (m, 2H), 6.04 (s, 1H), 7.54 (d, 1H), 7.66 (d, 1H)
R_{f} (Essigester): 0.68

### Schritt 4: 2,4-Dibrom-3-(2,2,2-trifluorethoxy)-cyclohexan-1,3-dion

0.340 g (0.70 mmol) ((3-Oxo-1-cyclohexenyl)-2,4-dibrom -3-(2,2,2-trifluorethoxy))-benzoat wurden in 10 ml Acetonitril gelöst. Es wurden 3 Tropfen Acetoncyanhydrin sowie 0.128 g (1.30 mmol) Triethylamin zugegeben. Die Mischung wurde 2 h bei RT gerührt, woraufhin 0.022 g (0.30 mmol) KCN zugegeben wurden. Nach weiteren 10 h bei RT wurde vollständig eingeengt, der Rückstand in Wasser aufgenommen und mit 6 N Salzsäure versetzt. Anschliessend wurde mit CH₂Cl₂ extrahiert. Nach Trocknen der organischen Phasen über Na₂SO₄, vollständigem Einengen und Chromatographie an Kieselgel (Laufmittel: Toluol/THF) erhielt man erhielt man 2,4-Dibrom-3-(2,2,2-trifluorethoxy)-cyclohexan-1,3-dion in Form eines farblosen Öls. Ausbeute: 0.143 g (42% der Theorie)
¹H-NMR: δ [CDCl3] 2.04 (m, 2H), 2.43 (m, 2H), 2.78 (d, 2H), 4.20 (m, 2H), 6.85 (2, 1 H), 7.59 (d, 1 H)
R_{f} (Essigester): 0.40

Die in nachfolgenden Tabellen aufgeführten Beispiele wurden analog oben genannten Methoden hergestellt beziehungsweise sind analog oben genannten Methoden erhältlich. Darin bedeuten die verwendeten Abkürzungen:
Et = Ethyl Me = Methyl Fp. = Festpunkt

**Tabelle A: Erfindungsgemäße Verbindungen der allgemeinen Formel (I), worin die Substituenten und Symbole folgende Bedeutungen haben:**

| | | | | |
|---|---|---|---|---|
| | R⁴ = OH | | n = 0 | |
| 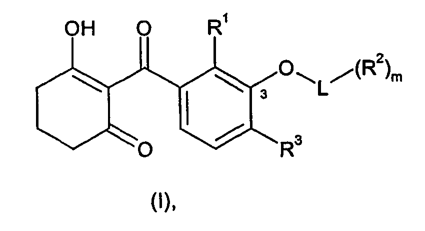 | | | | |

| **Nr.** | **R¹** | **-L-(R²)ₘ** | **R³** | **Physikal. Daten R_{f}(Essigester)** |
|---|---|---|---|---|
| 1.1 | Cl | OCH₂CF₂CF₂H | SO₂Et | 0,23 |
| 1.2 | Br | OCH₂CF₃ | Br | 0,40 |
| 1.3 | Cl | OCF₂H | Cl | |
| 1.4 | Cl | OCF₂(CHF)CF₃ | Cl | |
| 1.5 | Cl | OCH₂CF₂H | Cl | 0,45 |
| 1.6 | Cl | OCH₂CH₂Cl | Cl | |
| 1.7 | Cl | OCH₂CHCl₂ | Cl | |
| 1.8 | Cl | OCH₂CF₃ | Cl | 0,32 |
| 1.9 | Cl | OCF₂Cl | Cl | |
| 1.10 | Cl | OCF₂Br | Cl | |
| 1.11 | Cl | OCH₂CH₂CF₃ | Cl | 0,41 |
| 1.12 | Cl | OCF₂H | SO₂Me | |
| 1.13 | Br | OCF₂H | Br | |
| 1.14 | Cl | OCH₂CF₂CF₂H | Cl | 0,43 |
| 1.15 | Cl | OCH₂CCl₃ | Cl | |
| 1.16 | Cl | OCH₂C₃F₇ | Cl | 0,38 |
| 1.17 | Cl | OCH₂CCl₃ | SO₂Et | |
| 1.18 | Cl | OCH₂(CHCl)CH₂Cl | SO₂Et | |
| 1.19 | Br | OCH₂C₂F₅ | Br | 0,38 |
| 1.20 | Cl | OCH₂CF₂H | SO₂Me | |
| 1.21 | Cl | OCH₂CF₃ | SO₂Me | 0,07 |
| 1.22 | Cl | OCH₂CF₂CF₂H | SO₂Me | 0,16 |
| 1.23 | Cl | OCF₂(CHF)CF₃ | SO₂Et | |
| 1.24 | Cl | OCH₂C₃F₇ | SO₂Me | 0,29 |
| 1.25 | Cl | OCH₂CH₂CF₃ | SO₂Me | |
| 1.26 | Cl | OCF₂(CHF)CF₃ | SO₂Me | |
| 1.27 | Cl | OCH₂CH₂Cl | SO₂Me | |
| 1.28 | Cl | OCH₂CHCl₂ | SO₂Me | |
| 1.29 | Cl | OCH₂CCl₃ | SO₂Me | |
| 1.30 | Cl | OCH₂(CHCl)CH₂Cl | SO₂Me | |
| 1.31 | Cl | OCF₂Cl | SO₂Me | |
| 1.32 | Cl | OCH₂C₂F₅ | SO₂Me | 0,25 |
| 1.33 | Cl | OCH₂CH₂Cl | SO₂Et | |
| 1.34 | Cl | OCF₂H | SO₂Et | |
| 1.35 | Cl | OCH₂CF₂H | SO₂Et | 0,24 |
| 1.36 | Cl | OCH₂CHCl₂ | SO₂Et | |
| 1.37 | Br | OCF₂(CHF)CF₃ | Br | |
| 138 | Cl | OCH₂CF₃ | SO₂Et | 0,27 |
| 1.39 | Cl | OCH₂C₂F₅ | Cl | 0,45 |
| 1.40 | Br | OCH₂CH₂CF₃ | Br | |
| 1.41 | Cl | OCH₂CH₂CF₃ | SO₂Et | 0,53 |
| 1.42 | Br | OCH₂CH₂Cl | Br | |
| 1.43 | Br | OCH₂CF₂H | Br | 0,41 |
| 1.44 | Cl | OCH₂C₂F₅ | SO₂Et | 0,22 |
| 1.45 | Br | OCF₂Cl | Br | |
| 1.46 | Cl | OCF₂Cl | SO₂Et | |
| 1.47 | Cl | OCF₂Br | SO₂Et | |
| 1.48 | Cl | OCH₂CF₂Br | SO₂Et | |
| 1.49 | Cl | OCH₂C₃F₇ | SO₂Et | 0,23 |
| 1.51 | Br | OCH₂CHCl₂ | Br | |
| 1.52 | Br | OCH₂CCl₃ | Br | |
| 1.53 | Br | OCH₂C₃F₇ | Br | 0,29 |
| 1.54 | Br | OCH₂CF₂CF₂H | Br | 0,42 |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 Gew.-Teile | einer Verbindung der allgemeinen Formel(I), |
| 10 " | ligninsulfonsaures Calcium, |
| 5 " | Natriumlaurylsulfat, |
| 3 " | Polyvinyfalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 Gew.-Teile | einer Verbindung der allgemeinen Formel (I), |
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in einer in den Tabellen 1 bis 5 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse dieser Vergleichstabellen zeigen, weisen die ausgewählten erfindungsgemäßen Verbindungen dabei eine hervorragende Wirksamkeit gegen ein breites Spektrum wirtschaftlich wichtiger mono- und dikotyler Schadpflanzen auf.

### 2. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) und im Vergleich dazu mit den im Stand der Technik offenbarten erfolgt dann wie oben unter Punkt 1 beschrieben. Vier bis fünf Wochen nach der Applikation und Standzeit im Gewächshaus wird mittels optischer Bonitur festgestellt, daß die erfindungsgemäßen Verbindungen eine hervorragende Verträglichkeit gegenüber wichtigen Kulturpflanzen, insbesondere Weizen, Mais und Reis, aufweisen.

## Patentansprüche

1. Verbindungen der Formel (I) oder deren Salze 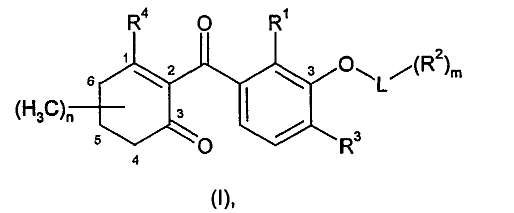 worin
L eine gesättigte Kohlenstoffkette mit 1, 2, 3 oder 4 Kohlenstoffatomen;
R¹ Iod, Brom, Chlor, Fluor, (C₁-C₄)-Alkyl, (C₁-C₄)-Alkylthio, (C₁-C₄)-Alkoxy oder (C₁-C₄)-Halogenalkyl;
R² Brom, Chlor, Iod oder Fluor;
R³ Brom, Chlor, Fluor, Cyano, Nitro, (C₁-C₄)-Alkyl, Methylsulfonyl oder Ethylsulfonyl;
R⁴ OR⁵ oder SR⁵;
R⁵ Wasserstoff, (C₁-C₄)-Alkyl, (C₂-C₄)-Alkenyl,(C₂-C₄)-Alkinyl, Benzyl, Phenyl;
m eine ganze Zahl von 1 bis 9 und
n 0, 1, oder 2 bedeuten,
mit der Maßgabe, daß
a) der Rest O - L - (R²)ₘ nicht für Trifluormethoxy stehen soll, und
b) die Verbindungen 2-(3-(2,3-Di-brompropoxy)-2-chlor-4-ethylsulfonylbenzoyl)-1,3-cyclohexandion und 2-(2-Chlor-3-(3-chlorpropoxy)-4-ethylsulfonylbenzoyl)-1,3-cyclohexandion
von obiger Definition nicht umfaßt sein sollen.

2. Verbindungen nach Anspruch 1, worin
R² Brom, Chlor oder Fluor bedeutet.

3. Verbindungen nach Anspruch 1 oder 2, worin
L eine gesättigte Kohlenstoffkette mit 1, 2 oder 3 Kohlenstoffatomen;
R¹ Brom, Chlor, Fluor, Methyl, Methylthio, Methoxy oder Trifluormethyl;
R³ Brom, Chlor, Fluor, Methylsulfonyl oder Ethylsulfonyl und
m eine ganze Zahl von 1 bis 7 bedeuten.

4. Verbindungen nach einem der Ansprüche 1 bis 3, worin
R² Chlor oder Fluor und
R³ Chlor, Fluor, Methylsulfonyl oder Ethylsulfonyl bedeuten.

5. Verbindungen nach einem der Ansprüche 1 bis 4, worin
R¹ Brom oder Chlor;
R⁵ Wasserstoff und
n 0 bedeuten.

6. Verbindungen nach einem der Ansprüche 1 bis 5, worin R¹ und R³ dieselbe Bedeutung haben.

7. Verbindungen nach einem der Ansprüche 1 bis 6, worin R¹ für Chlor steht.

8. Herbizide Mittel, **gekennzeichnet durch** einen herbizid wirksamen Gehalt an mindestens einer Verbindung der allgemeinen Formel (I) gemäß einem der Ansprüche 1 bis 7.

9. Herbizide Mittel nach Anspruch 8 in Mischung mit Formulierungshilfsmitteln.

10. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 7 oder eines herbiziden Mittels nach Anspruch 8 oder 9 auf die Pflanzen oder auf den Ort des unerwünschten Pflanzenwachstums appliziert.

11. Verwendung von Verbindungen der allgemeinen Formel (I) nach einem der Ansprüche 1 bis 7 oder von herbiziden Mitteln nach Anspruch 8 oder 9 zur Bekämpfung unerwünschter Pflanzen.

12. Verwendung nach Anspruch 11, **dadurch gekennzeichnet, daß** die Verbindungen der allgemeinen Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. A compound of the formula (I) or salt thereof 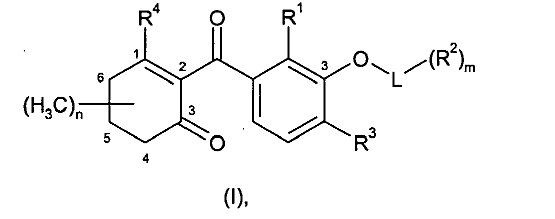 in which
L is a saturated carbon chain having 1, 2, 3 or 4 carbon atoms;
R¹ is iodine, bromine, chlorine, fluorine, C₁-C₄ alkyl, C₁-C₄ alkylthio, C₁-C₄ alkoxy or C₁-C₄ haloalkyl;
R² is bromine, chlorine, iodine or fluorine;
R³ is bromine, chlorine, fluorine, cyano, nitro, C₁-C₄ alkyl, methylsulfonyl or ethylsulfonyl;
R⁴ is OR⁵ or SR⁵;
R⁵ is hydrogen, C₁-C₄ alkyl, C₂-C₄ alkenyl, C₂-C₄ alkynyl, benzyl or phenyl;
m is an integer from 1 to 9, and
n is 0, 1 or 2,
with the proviso that
a) the radical O - L - (R²)ₘ is not to be trifluoromethoxy, and
b) the compounds 2-(3-(2,3-dibromopropoxy)-2-chloro-4-ethylsulfonylbenzoyl)-1,3-cyclohexanedione and 2-(2-chloro-3-(3-chloropropoxy)-4-ethylsulfonyl-benzoyl)-1,3-cyclohexanedione
are to be excluded from the above definition.

2. A compound as claimed in claim 1, wherein
R² is bromine, chlorine or fluorine.

3. A compound as claimed in claim 1 or 2, wherein
L is a saturated carbon chain having 1, 2 or 3 carbon atoms;
R¹ is bromine, chlorine, fluorine, methyl, methylthio, methoxy or trifluoromethyl;
R³ is bromine, chlorine, fluorine, methylsulfonyl or ethylsulfonyl, and
m is an integer from 1 to 7.

4. A compound as claimed in any one of claims 1 to 3, wherein
R² is chlorine or fluorine and
R³ is chlorine, fluorine, methylsulfonyl or ethylsulfonyl.

5. A compound as claimed in any one of claims 1 to 4, wherein
R¹ is bromine or chlorine;
R⁵ is hydrogen, and
n is 0.

6. A compound as claimed in any one of claims 1 to 5, wherein R¹ and R³ have the same definition.

7. A compound as claimed in any one of claims 1 to 6, wherein R¹ is chlorine.

8. A herbicidal composition comprising a herbicidally active amount of at least one compound of the formula (I) as claimed in any one of claims 1 to 7.

9. A herbicidal composition as claimed in claim 8 in a mixture with formulating auxiliaries.

10. A method of controlling unwanted plants which comprises applying an effective amount of at least one compound of the formula (I) as claimed in any one of claims 1 to 7 or of a herbicidal composition as claimed in claim 8 or 9 to the plants or to the site of the unwanted plant growth.

11. The use of a compound of the formula (I) as claimed in any one of claims 1 to 7 or of a herbicidal composition as claimed in claim 8 or 9 to control unwanted plants.

12. The use as claimed in claim 11, wherein the compounds of the formula (I) are used to control unwanted plants in crops of useful plants.

13. The use as claimed in claim 12, wherein the useful plants are transgenic plants.

## Revendications

1. Composés de formule (I) ou leurs sels 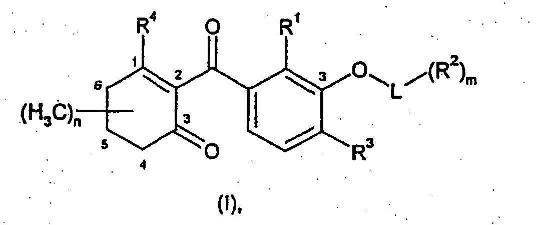 où
L représente une chaîne carbonée saturée présentant 1, 2, 3 ou 4 atomes de carbone ;
R¹ représente un atome d'iode, de brome, de chloré, de fluor, un groupe alkyle en C₁-C₄, (alkyl en C₁-C₄)thio, alkoxy en C₁-C₄ ou halogénoalkyle en C₁-C₄ ;
R² représente un atome de brome, de chlore, d'iode ou de fluor ;
R³ représente un atome de brome, de chlore, de fluor, un groupe cyano, nitro, alkyle en C₁-C₄, méthylsulfonyle ou éthylsulfonyle ;
R⁴ représente un groupe OR⁵ ou SR⁵ ;
R⁵ représente un atome d'hydrogène, un groupe alkyle en C₁-C₄, alcényle en C₂-C₄, alcynyle en C₂-C₄, benzyle, phényle ;
m est un entier de 1 à 9 et
n vaut 0, 1 ou 2,
à condition que
a) le reste O-L-(R²)ₘ ne représente pas le groupe trifluorométhoxy, et
b) les composés 2-(3-(2,3-di-bromopropoxy)-2-chloro-4-éthylsulfonylbenzoyl)-1,3-cyclohexandione et 2-(2-chloro-3-(3-chloropropoxy)-4-éthylsulfonyl-benzoyl)-1,3-cyclohexanedione, ne soient pas compris par la définition ci-dessus.

2. Composés selon la revendication 1, où
R² représente un atome de brome, de chlore ou de fluor.

3. Composés selon la revendication 1 ou 2, où
L représente une chaîne carbonée saturée présentant 1, 2 ou 3 atomes de carbone ;
R¹ représente un atome de brome, de chlore, de fluor, un groupe méthyle, méthylthio, méthoxy ou trifluorméthyle ;
R³ représente un atome de brome, de chlore, de fluor, un groupe méthylsulfonyle ou éthylsulfonyle, et
m est un entier de 1 à 7.

4. Composés selon l'une des revendications 1 à 3, où
R² représente un atome de chlore ou de fluor, et
R³ représente un atome de chlore, de fluor, un groupe méthylsulfonyle ou éthylsulfonyle.

5. Composés selon l'une des revendications 1 à 4, où
R¹ représente un atome de brome ou de chlore ;
R⁵ représente un atome d'hydrogène et
n vaut 0.

6. Composés selon l'une des revendications 1 à 5, où R¹ et R³ possèdent la même signification.

7. Composés selon l'une des revendications 1 à 6, où R¹ représente un atome de chlore.

8. Agents herbicides **caractérisés en ce qu'**ils renferment une teneur à efficacité herbicide d'au moins un composé de formule générale (I) selon l'une des revendications 1 à 7.

9. Agents herbicides selon la revendication 8 en mélange avec des formulants.

10. Procédé pour la lutte contre les plantes indésirables, **caractérisé en ce qu'**on applique une quantité efficace d'au moins un composé de formule générale (I) selon l'une des revendications 1 à 7 ou d'un agent herbicide selon la revendication 8 ou 9 aux plantes ou à l'endroit de croissance de la végétation indésirable.

11. Utilisation de composés de formule générale (I) selon l'une des revendications 1 à 7 ou d'agents herbicides selon la revendication 8 ou 9, dans la lutte contre les plantes indésirables.

12. Utilisation selon la revendication 11, **caractérisée en ce qu'**on utilise les composés de formule générale (I) dans la lutte contre une végétation indésirable dans les cultures de plantes utiles.

13. Utilisation selon la revendication 12, **caractérisée en ce que** les plantes utiles sont des plantes utiles transgéniques.
